# EUROPÄISCHE PATENTSCHRIFT

(11) **EP 0 353 531 B1**
(45) Veröffentlichungstag und Bekanntmachung des Hinweises auf die Patenterteilung: **21.04.1993**
(21) Anmeldenummer: 89113099.9
(22) Anmeldetag: 17.07.1989
(51) Int. Cl.: C09B 51/00, A61K 7/13

(54) **Haarfärbemittel**
Hair dye
Teinture capillaire

(30) Priorität: 25.07.1988 DE 3825212
(43) Veröffentlichungstag der Anmeldung: 07.02.1990
(73) Patentinhaber: Henkel Kommanditgesellschaft auf Aktien, 40191 Düsseldorf (DE)
(72) Erfinder: Rose, David, Dr., D-4010 Hilden (DE); Lieske, Edgar, D-4000 Düsseldorf (DE); Höffkes, Horst, Dr., D-4000 Düsseldorf-Hellerhof (DE)

(56) Entgegenhaltungen:
- DE-A- 1 804 066
- US-A- 2 196 739
- JOURNAL OF HETEROCYCLIC CHEM., Band 10, 1973, Seiten 213-215; K.V. RAO et al.: "Reaction of sodium borohydride with heteroaromatic nitro compounds"

## Beschreibung

Gegenstand der Erfindung sind Haarfärbemittel mit einem Gehalt an direktziehenden Haarfarbstoffen.

Für das Färben von Haar spielen neben den Oxidationsfarbstoffen, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Üblicherweise enthalten Haarfärbemittel solche direktziehende Haarfarbstoffe in einem kosmetischen Träger. Als kosmetische Träger für die direktziehenden Haarfarbstoffe und die zur Erzielung bestimmter Nuancen gegebenenfalls im Haarfärbemittel zusätzlich enthaltenen Oxidationsfarbstoffvorprodukte dienen Cremes, Emulsionen, Gele, Shampoos, Schaumaerosole oder andere Zubereitungen, die für die Anwendung auf Haar geeignet sind.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabillität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Direktziehende Farbstoffe sollen weiterhin eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Nachteile der bekannten direktziehenden Nitroanilin-Farbstoffe sind jedoch einerseits die begrenzte Wasserlöslichkeit, die zu Problemen bei der Formulierung der Haarfärbemittel fürht, andererseits die unbefriedigende Waschechtheit, d. h. daß die Haarfärbungen nach mehrmaligem Haarwaschen stark ausbluten. Weiterhin bereitet es Schwierigkeiten, bei Verwendung direktziehender Farbstoffe bestimmte modische Farbtöne, insbesondere rote Farbtöne, zu erzielen. Bisher werden dafür in der Regel 2-Nitro-p-phenylendiamin und dessen amino-substituierten Derivate verwendet. Die meisten dieser Verbindungen sind jedoch in Wasser nur unzureichend löslich oder dispergierbar. Dies führt häufig zu ungleichmäßigen Färbungen. Insbesondere bei Färbemitteln, die zur Erzielung bestimmter Nuancen hohe Konzentration an Farbstoffen enthalten, aber auch bei Zubereitungen mit nur wenig solubilisierenden Träger-Komponenten besteht weiterhin die Gefahr, daß schwächere Färbungen als vorgesehen erhalten werden, weil die Farbstoffe auskristallisieren und im Färbebad verbleiben, anstatt auf die Haare aufzuziehen.

Es wurde überraschenderweise gefunden, daß bestimmte 1,2,3,4-Tetrahydro-nitrochinoxaline diese genannten Nachteile nicht besitzen und daher die an direktziehende Haarfarbstoffe gestellten Anforderungen in besonderem Maße erfüllen.

Gegenstand der Erfindung sind daher Haarfärbemittel mit einem Gehalt an direktziehenden Farbstoffen, dadurch gekennzeichnet, daß als direktziehende Farbstoffe eine oder mehrere Verbindungen der Formel (I), 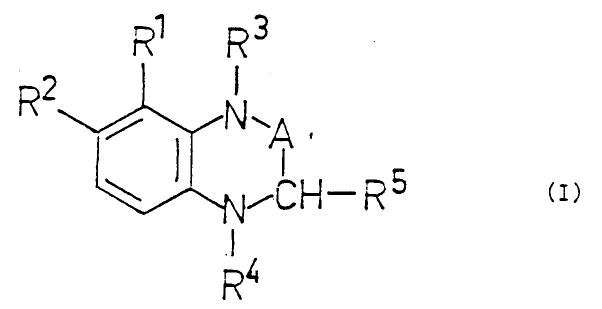
in der eine der Gruppen R¹ oder R² eine Nitrogruppe ist, während die zweite Gruppe Wasserstoff, Halogen, eine Aminogruppe, eine Mono- oder Dialkylaminogruppe mit Alkylgruppen mit 1 bis 4 Kohlenstoffatomen, eine Alkyl- oder Alkoxygruppe, jeweils mit 1 bis 4 Kohlenstoffatomen, ist, R³ und R⁴ unabhängig voneinander Wasserstoff oder eine Alkylgruppe mit 1 bis 4 Kohlenstoffatomen sind, A eine >CHR⁶- oder eine >C=O-Gruppe ist, wobei R⁶ Wasserstoff oder eine Alkylgruppe mit 1 bis 4 Kohlenstoffatomen ist, und R⁵ Wasserstoff oder eine Alkylgruppe mit 1 bis 4 Kohlenstoffatomen darstellt, oder deren Salze enthalten sind.

Besonders gut geeignete Farbstoffe sind 1,2,3,4-Tetrahydro-5-nitrochinoxalin und 1,2,3,4-Tetrahydro-6-nitrochinoxalin.

Die erfindungsgemäßen Farbstoffe sind in der Regel nach gängigen Syntheseverfahren aus den entsprechend substituierten Chinoxalinen zugänglich. So wird die Herstellung von 1,2,3,4-Tetrahydro-5-nitrochinoxalin und 1,2,3,4-Tetrahydro-6-nitrochinoxalin durch Umsetzung der entsprechenden Chinoxaline mit Natriumborhydrid in methanolischer Lösung im J. Het. Chem. 10, 213 (1973) beschrieben. Verbindungen der Formel (I) sind aber auch auf anderen Wegen zugänglich. So ist 1,2,3,4-Tetrahydro-7-nitro-2-oxochinoxalin nach J. Chem. Soc. (1961), 5283 durch Umsetzung von N-(2,4-dinitrophenyl)glycin mit einer Mischung aus Natriumhydrogenocarbonat und Natriumsulfid in methanolischer Lösung zugänglich.

Den genannten Veröffentlichungen sind aber keine Hinweise auf eine Eignung dieser Verbindungen zum Färben von Haaren zu entnehmen.

Neu und daher ein weiterer Gegenstand der Erfindung sind Verbindungen der Formel (I) gemäß Anspruch 1, gekennzeichnet durch die folgenden Substituentenkombinationen (I) bis (IV):

| | R¹ | R² | R³ | R⁴ | R⁵ | A |
|---|---|---|---|---|---|---|
| I: | H | NO₂ | H | CH₃ | H | CH₂ |
| II: | H | NO₂ | CH₃ | CH₃ | H | CH₂ |
| III: | NH₂ | NO₂ | H | H | H | CH₂ |
| IV: | H | NO₂ | H | H | CH₃ | CH₂ |

Diese Verbindungen sind ebenfalls prinzipiell aus den entsprechend substituierten Chinoxalinen zugänglich; Synthesevorschriften sind im Beispielteil angegeben.

Die Farbstoffe der allgemeinen Formel (I) erzeugen auf dem Haar gelbe bis rotorange Farbnuancen von hoher Intensität, Licht- und Waschechtheit. Sie besitzen darüber hinaus im Vergleich zu bekannten Nitroanilinfarbstoffen eine bessere Löslichkeit in wäßrig-alkalischen Medien. In dermatologischer und toxikologischer Hinsicht sind die genannten Verbindung unschädlich und daher für die Anwendung in Haarfärbemitteln besonders geeignet.

Die erfindungsgemäßen Farbstoffe können sowohl selbst als auch in Form ihrer wasserlöslichen Salze eingesetzt werden. Salze werden in diesem Zusammenhang dann als wasserlöslich angesehen, wenn sie sich in einer zum Zwecke des Haarefärbens ausreichenden Menge in Wasser oder wäßrigen Mischungen lösen.

Unter den wasserlöslichen Salzen sind in erster Linie die Salze starket Basen wie etwa die Alkalisalze, z. B. das Natrium- oder Kaliumsalz oder Ammoniumsalze und Alkanolammoniumsalze mit 2 bis 4 C-Atomen in der Alkanolgruppe wie z. B. das Monoethanolammoniumsalz, das Triethanolammoniumsalz oder das Isopropanolammoniumsalz zu verstehen.

Die erfindungsgemäßen Haarfärbemittel können die direktziehenden Verbindungen gemäß Formel (I) allein oder in Kombination mit bekannten direktziehenden Farbstoffen, z. B. mit anderen Nitrobenzolderivaten, Anthrachinonfarbstoffen, Triphenylmethan- oder Azofarbstoffen enthalten. Darüber hinaus eignen sich die direktziehenden Farbstoffe gemäß Formel (I) wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdikkungsmittel wie z. B. Methyl- oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,01 bis 5 Gew.-%, vorzugsweise von 1 bis 3 Gew.-%, enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit dem erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

### Beispiele

### Herstellungsbeispiele

### 1.) 1,2,3,4-Tetrahydro-2-methyl-6-nitrochinoxalin

1 g 2-Methyl-6-nitrochinoxalin, hergestellt gemäß der in Coll. Czech. Chem. Comm. 30, 3106 (1965) beschriebenen Synthesevorschrift aus 4-Nitro-1,2-diaminobenzol durch Umsetzung mit Methylglyoxal, wurden in 30 ml Essigsäure gelöst. Zu dieser Lösung wurde bei +5 °C solange in kleinen Portionen Natriumborhydrid gegeben, bis die Ausgangsverbindung in der Lösung dünnschichtchromatographisch nicht mehr nachweisbar war. Die Lösung wurde sodann mit 200 ml Wasser verdünnt und zweimal mit Chloroform extrahiert. Die organische Phase wurde mit Wasser nachgewaschen und getrocknet. Nach dem Einengen zur Trockne wurde der Rückstand in Aceton gelöst und mit Salzsäure gefällt. Die Ausbeute betrug 0,6 g (58 % d.Th.). Der Schmelzpunkt des so erhaltenen 1,2,3,4-Tetrahydro-2-methyl-6-nitrochinoxalin lag bei 178 °C.

### 2.) 1,2,3,4-Tetrahydro-5-amino-6-nitrochinoxalin

Nach der in J.Chem.Soc., Perkin I, 1975, 1229 publizierten Synthesevorschrift wurde 5-Amino-6-nitrochinoxalin durch Umsetzung von 6-Nitrochinoxalin mit Hydroxylamin in einem alkalischen Medium hergestellt. Das Produkt wurde analog Beispiel 1) mit Natriumborhydrid umgesetzt. Das in einer Ausbeute von 11,5 % d. Th. als braune Kristalle erhaltene 1,2,3,4-Tetrahydro-5-amino-6-nitrochinoxalin hatte einen Schmelzpunkt von 213 °C.

### 3.) N,N′-Dimethyl-1,2,3,4-tetrahydro-6-nitrochinoxalin

Zu einer Mischung von 1,08 g (0,006 mol) des nach der Vorschrift in J. Het. Chem. 10, 213 (1973) erhaltenen 1,2,3,4-Tetrahydro-6-nitro-chinoxalins und 1,5 g (0,005 mol) Paraformaldehyd in 300 ml Essigsäure wurden bei 25 °C 1,52 g (0,024 mol) Natriumcyanborhydrid zugegeben. Danach wurde die Lösung 24 Stunden bei Raumtemperatur belassen. Anschließend wurde die Lösung in 60 ml 25 %ige wäßrige NaOH-Lösung gegossen. Die erhaltene Mischung wurde dann dreimal mit Methylenchlorid extrahiert. Nach dem Trocknen und Einengen wurden 0,35 g ( 35 % d. Th.) an N,N′-Dimethyl-1,2,3,4-tetrahydro-6-nitrochinoxalin erhalten. Der Schmelzpunkt des Produktes betrug 70 °C.

### 4.) 1-(N-Methyl)-1,2,3,4-tetrahydro-6-nitrochinoxalin

### Stufe 1:

Eine Mischung von 21,75 g (0,14 mol) 2-Fluor-5-nitroanilin, 56 g (0,74 mol) 2-Methylamino-ethanol und 1 g Kupfer(II)oxid wurden 1,5 Stunden auf 120 °C erhitzt. Nach dem Abkühlen wurde der Katalysator abfiltriert und die Lösung auf Eiswasser gegossen. Der entstandene Niederschlag wurde abgesaugt und im Vakuum bei + 70 °C getrocknet. Die Ausbeute an 1-(N-Methyl-N-ß-hydroxyethyl)-2-amino-4-nitrobenzol betrug 25,5 g (96 % d. Th.). Der Schmelzpunkt des Produktes lag bei 111 bis 113 °C.

### Stufe 2:

5 g des Produktes aus Stufe 1 wurden innerhalb von 15 Minuten in 22 ml Phosphoroxidtrichlorid bei Raumtemperatur eingetragen. Anschließend wurde die Mischung 1 Stunde auf dem Wasserbad erwärmt. Nach dem Abdestillieren des überschüssigen Phosphoroxidtrichlorids wurde der Rückstand mit 50 ml destilliertem Wasser versetzt. Anschließend wurde mit Diethylether extrahiert. Das durch Einengen der etherischen Phase erhaltene, orange-farbige Öl wurde mit Ethanol 16 Stunden lang unter Rückfluß erhitzt und im Vakuum bis zur Trockne eingeengt. Der erhaltene Rückstand wurde in Wasser gelöst und mit einer Kaliumcarbonat-Lösung versetzt, bis die Mischung alkalisch reagierte. Der dabei entstehende Niederschlag wurde abgesaugt und im Vakuum bei 60 °C getrocknet. Es wurden orange-rote Kristalle von 1-(N-Methyl)-1,2,3,4-tetrahydro-6-nitrochinoxalin erhalten. Das mit Ethanol als Lösungsmittel aufgenommene UV-Spektrum dieser Kristalle zeigte Maxima bei 441 nm, 350 nm und 290 nm.

### Haarfärbeversuche

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄ + 2 EO-sulfat, Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Das Ergebnis der Färbeversuche ist der Tabelle I zu entnehmen.

**Tabelle I**

| direktziehender Farbstoff | Nuance des gefärbten Haares |
|---|---|
| 1,2,3,4-Tetrahydro-5-nitrochinoxalin | graurot |
| 1,2,3,4-Tetrahydro-6-nitrochinoxalin | kupfer |
| 1,2,3,4-Tetrahydro-7-nitro-2-oxochinoxalin | olivbraun |
| 1,2,3,4-Tetrahydro-2-methyl-6-nitrochinoxalin | rotgold |
| 1,2,3,4-Tetrahydro-5-amino-6-nitrochinoxalin | gelb |
| N,N′-Dimethyl-1,2,3,4-tetrahydro-6-nitrochinoxalin | rotorange |
| 1-(N-Methyl)-1,2,3,4-tetrahydro-6-nitrochinoxalin | rotbraun |

## Patentansprüche

1. Haarfärbemittel mit einem Gehalt an direktziehenden Farbstoffen, dadurch gekennzeichnet, daß als direktziehende Farbstoffe eine oder mehrere Verbindungen der Formel (I), 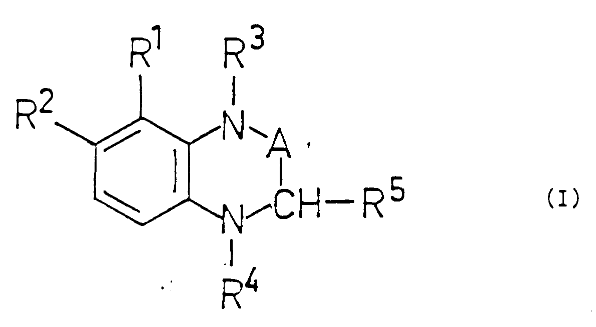 in der eine der Gruppen R¹ oder R² eine Nitrogruppe ist, während die zweite Gruppe Wasserstoff, Halogen, eine Aminogruppe, eine Mono- oder Dialkylaminogruppe mit Alkylgruppen mit 1 bis 4 Kohlenstoffatomen, eine Alkyl- oder Alkoxygruppe, jeweils mit 1 bis 4 Kohlenstoffatomen, ist, R³ und R⁴ unabhängig voneinander Wasserstoff oder eine Alkylgruppe mit 1 bis 4 Kohlenstoffatomen sind, A eine >CHR⁶- oder eine >C=O-Gruppe ist, wobei R⁶ Wasserstoff oder eine Alkylgruppe mit 1 bis 4 Kohlenstoffatomen ist, und R⁵ Wasserstoff oder eine Alkylgruppe mit 1 bis 4 Kohlenstoffatomen darstellt, oder deren Salze enthält.

2. Haarfärbemittel nach Anspruch 1, dadurch gekennzeichnet, daß als direktziehende Farbstoffe 1,2,3,4-Tetrahydro-5-nitrochinoxalin und/oder 1,2,3,4-Tetrahydro-6-nitrochinoxalin enthalten sind.

3. Haarfärbemittel nach einem der Ansprüche 1 oder 2, dadurch gekennzeichnet, daß die direktziehenden Farbstoffe gemäß Formel (I) in einer Menge von 0,01 bis 5 Gew.-%, insbesondere von 0,1 bis 2 Gew.-%, bezogen auf das gesamte Haarfärbemit tel, enthalten sind.

4. Haarfärbemittel nach einem der Ansprüche 1 bis 3, dadurch gekennzeichnet, daß zusätzlich zu den Verbindungen der Formel (I) noch weitere direktziehende Haarfarbstoffe und/oder Oxidationsfarbstoffvorprodukte enthalten sind.

5. Haarfärbemittel nach einem der Ansprüche 1 bis 4, dadurch gekennzeichnet, daß diese Farbstoffe in einen kosmetischen Träger in Form einer Creme, einer Emulsion, eines Gels, eines Shampoos oder eines Schaumaerosols eingearbeitet sind.

6. Verbindungen der Formel (I) gemäß Anspruch 1, gekennzeichnet durch die folgenden Substituentenkombinationen (I) bis (IV):
| | R¹ | R² | R³ | R⁴ | R⁵ | A |
|---|---|---|---|---|---|---|
| I: | H | NO₂ | H | CH₃ | H | CH₂ |
| II: | H | NO₂ | CH₃ | CH₃ | H | CH₂ |
| III: | NH₂ | NO₂ | H | H | H | CH₂ |
| IV: | H | NO₂ | H | H | CH₃ | CH₂ |

## Claims

1. Hair-dyeing preparations containing substantive dyes which are characterized in that they contain one or more compounds corresponding to formula (I) 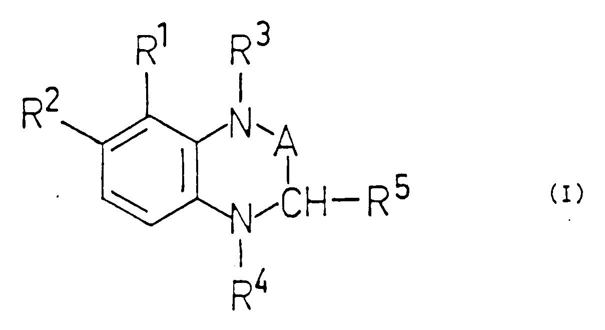 in which one of the substituents R¹ or R² is a nitro group while the other is hydrogen, halogen, an amino group, a mono- or dialkylamino group containing C₁₋₄ alkyl groups, a C₁₋₄ alkyl or alkoxy group, R³ and R⁴ independently of one another represent hydrogen or a C₁₋₄ alkyl group, A is a >CHR⁶- or a >C=O- group, where R⁶ is hydrogen or a C₁₋₄ alkyl group, and R⁵ is hydrogen or a C₁₋₄ alkyl group,
or salts thereof as the substantive dyes.

2. Hair-dyeing preparations as claimed in claim 1, characterized in that they contain 1,2,3,4-tetrahydro-5-nitroquinoxaline and/or 1,2,3,4-tetrahydro-6-nitroquinoxaline as the substantive dyes.

3. Hair-dyeing preparations as claimed in claim 1 or 2, characterized in that the substantive dyes corresponding to formula (I) are present in a quantity of 0.01 to 5% by weight and more particularly in a quantity of 0.1 to 2% by weight, based on the hair-dyeing preparation as a whole.

4. Hair-dyeing preparations as claimed in any of claims 1 to 3, characterized in that other substantive dyes and/or oxidation dye precursors are present in addition to the compounds of formula (I).

5. Hair-dyeing preparations as claimed in any of claims 1 to 4, characterized in that the dyes are incorporated in a cosmetic carrier in the form of a cream, an emulsion, a gel, a shampoo or a foam aerosol.

6. Compounds corresponding to formula (I) in claim 1, characterized by the following substituent combinations (I) to (IV):
| | R¹ | R² | R³ | R⁴ | R⁵ | A |
|---|---|---|---|---|---|---|
| I: | H | NO₂ | H | CH₃ | H | CH₂ |
| II: | H | NO₂ | CH₃ | CH₃ | H | CH₂ |
| III: | NH₂ | NO₂ | H | H | H | CH₂ |
| IV: | H | NO₂ | H | H | CH₃ | CH₂ |

## Revendications

1. Agents capillaires de coloration contenant des colorants montant directement sur la fibre, caractérisés en ce que, comme colorants montant directement sur la fibre, ils contiennent un ou plusieurs composés répondant à la formule (I) 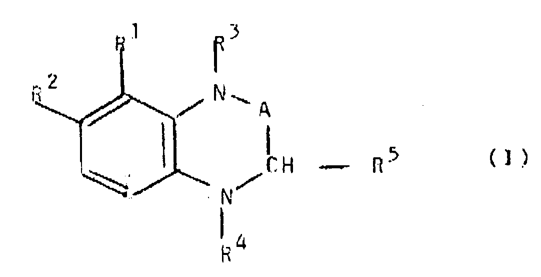 dans laquelle un des groupes R¹ ou R² représente un groupe nitro, tandis que le deuxième groupe représente un atome d'hydrogène, un atome d'halogène, un groupe amino, un groupe mono ou dialkylamino dont les groupes alkyle contiennent de 1 à 4 atomes de carbone, un groupe alkyle ou alcoxy contenant chacun de 1 à 4 atomes de carbone, R³ et R⁴ représentent, indépendamment l'un de l'autre, un atome d'hydrogène ou un groupe alkyle contenant de 1 à 4 atomes de carbone, A représente un groupe >CHR⁶- ou un groupe >C=O-, R⁶ représentant un atome d'hydrogène ou un groupe alkyle contenant de 1 à 4 atomes do carbone et R⁵ représentant un atome d'hydrogène ou un groupe alkyle contenant de 1 à 4 atomes de carbone, ou encore leurs sels.

2. Agents capillaires de coloration selon la revendication 1, caractérisés en ce que, comme colorants montant directement sur la fibre, ils contiennent la 1,2,3,4-tétrahydro-5-nitroquinoxaline et/ou la 1,2,3,4-tétrahydro-6-nitroquinoxaline.

3. Agents capillaires de coloration selon la revendication 1 ou 2, caractérisés en ce qu'ils contiennent les colorants montant directement sur la fibre selon la formule (I), en une quantité de 0,01 à 5% en poids, en particulier de 0,1 à 2% en poids, rapportée à l'agent capillaire de coloration totale.

4. Agents capillaires de coloration selon l'une quelconque des revendications 1 à 3, caractérisés en ce qu'il contiennent, on plus des composés de formule (I), encore d'autres colorants capillaires montant directement sur la fibre et/ou des précurseurs de colorants d'oxydation.

5. Agents capillaires de coloration selon l'une quelconque des revendications 1 à 4, caractérisés en ce que ces colorants sont incorporés dans un support cosmétique sous forme d'une crème, d'une émulsion, d'un gel, d'un shampoing ou d'un aérosol en mousse.

6. Composés de formule (I) salon la revendication 1, caractérisés par les combinaisons de substituants ci-après (I) à (IV) :
| | R¹ | R² | R³ | R⁴ | R⁵ | A |
|---|---|---|---|---|---|---|
| I : | H | NO₂ | H | CH₃ | H | CH₂ |
| II : | H | NO₂ | CH₃ | CH₃ | H | CH₂ |
| III : | NH₂ | NO₂ | H | H | H | CH₂ |
| IV : | H | NO₂ | H | H | CH₃ | CH₂ |
